# EUROPEAN PATENT SPECIFICATION

(11) **EP 0 815 288 B1**
(45) Date of publication and mention of the grant of the patent: **16.08.2006**
(21) Application number: 96908852.5
(22) Date of filing: 18.03.1996
(51) Int. Cl.: C23F 11/14, C07D 249/18

(54) **METHODS OF INHIBITING CORROSION USING N-HALO-AZOLES**
VERFAHREN ZUR KORROSIONSINHIBIERUNG UNTER VERWENDUNG VON N-HALO-AZOLEN
PROCEDES D'INHIBITION DE LA CORROSION AU MOYEN DE N-HALO-AZOLES

(30) Priority: 21.03.1995 US 407173
(43) Date of publication of application: 07.01.1998
(73) Proprietor: BetzDearborn Inc, Trevose, PA 19053-6783 (US)
(72) Inventor: REICHGOTT, David, M., Seattle, WA 98177 (US); ANDERSON, Sydia, B., Doylestown, PA 18901 (US); CADY, Michael, A., Yardley, PA 19067 (US); MAY, Roger, C., Glenside, PA 19038 (US); MONINO, Anita, G., Horsham, PA 19044 (US)
(74) Representative: W.P. Thompson & Co.
(86) International application number: PCT/US1996/003659
(87) International publication number: WO 1996/029449

(56) References cited:
- EP-A- 0 256 978
- EP-A- 0 592 118
- US-A- 4 184 991
- CORROSION, vol. 41, no. 1, January 1985, HOUSTON US, pages 39-45, XP002008378 HOLLANDER O.: "The chemistry of azole copper corrosion inhibitors in cooling water" cited in the application
- CORROSION, vol. 50, no. 6, June 1994, HOUSTON US, pages 422-431, XP000457454 LU F.: "Effect of halogenation on yellow metal corrosion : inhibition by triazoles" cited in the application
- DATABASE WPI Section Ch, Week 197417, Derwent Publications Ltd., London, GB; Class E13, AN 1974-31998V & JP 49 014 056 B (MATSUI SHIKISO KAGAKU) 04 April 1974
- WERKSTOFFE UND KORROSION, vol. 30, no. 10, October 1979 (1979-10), page 740, WEINHEIM, DE
- REES C.W.: '1-Chlorobenzotriazole: a new oxidant' JOURNAL OF THE CHEMICAL SOCIETY vol. C, 1969, pages 1474 - 1477

## Description

### FIELD OF THE INVENTION

The present invention relates to the inhibition of corrosion of steel and copper alloys in an aqueous system being treated with a halogen.

### BACKGROUND OF THE INVENTION

The use of triazoles for inhibiting the corrosion of copper and iron alloys in a wide variety of aqueous and non-aqueous systems is well known. In industrial cooling water systems, benzotriazole and tolyltriazole are used most often. Tolyltriazole is generally preferred because of its lower cost. Triazoles are film forming materials that provide efficient j coverage of metal or metal oxide surfaces in a system thereby providing protection against corrosive elements present in an aqueous system. In addition to the film forming tendency of various azoles, they also precipitate soluble, divalent copper ions. The precipitation prevents transport of copper ions to ferrous surfaces, where galvanic reactions between copper ions and iron atoms leads to pitting corrosion of the ferrous metal.

While the use of azoles for corrosion inhibition is widespread, there are drawbacks to their use, specifically with tolyltriazole. The most important drawbacks are experienced when azoles are used in combination with oxidizing halogens. Oxidizing halogens such as elemental chlorine, bromine, their hypohalous acids, or their alkaline solutions (i.e., solutions of hypochlorite or hypobromite ion) are the most common materials used to control microbiological growth in cooling water systems. When copper or iron alloys that have previously been protected with azoles are exposed to an oxidizing halogen, corrosion protection breaks down. After breakdown, it is difficult to form new protective films in tolyltriazole treated cooling systems that are being chlorinated, particularly continuously chlorinated. Very high dosages of tolyltriazole are frequently applied in an attempt to improve performance, often with limited success.

The degradation of protection of azole films in the presence of oxidizing halogens is well-documented in the literature. For example, R. Holm, et al., concluded that hypochlorite penetrates an intact triazole film, leading to higher corrosion rates, and that secondly, hypochlorite attacks the prefilmed triazole surface, disrupting or degrading the film (53rd Annual Meeting of the International Water Conference, Paper No. IWC-92-40, 1992). Lu, et al., also studied interactions of triazole films with hypochlorite on copper and copper alloy surfaces ("Effects of Halogenation on Yellow Metal Corrosion: Inhibition by Triazoles", Corrosion, 50, 422 (1994)). Lu, et al., concluded:
(a) prefilmed tolyltriazole on copper and brass surfaces undergoes decomposition during chlorination;
(b) the stability of prefilmed tolyltriazole on copper and brass to NaOCI was improved when tolyltriazole was added to the hypochlorite solution;
© clean (i.e. non-prefilmed) copper surfaces did not develop good protective films when placed in solutions containing mixtures of tolyltriazole and NaOCI.

Thus, the combination of tolyltriazole with NaOCI did not produce a composition capable of efficient film formation and corrosion inhibition.

European Patent Application 0 592 118 discloses a method of controlling corrosion and biological matter in copper and copper alloy cooling water systems. The method requires that a triazole film be formed on copper and copper alloys by treating the system with a triazole. Thereafter, when needed, low levels of triazole and an oxidizing biocide are added to the system at the same time. Re-formation of the triazole film is accomplished by adding additional triazole when required. This method does not involve the ex situ reaction of a halogenating agent and a benzotriazole to form a halo-benzotriazole treatment.

US-A-4184991 discloses a corrosion inhibiting composition for ferrous metals in a cooling water system. The composition comprises an admixture of a benzotriazole, a tolyltriazole, a substituted benzotriazole or a substituted tolyltriazole, with an acrylic or methacrylic acid ester polymer. There is however no disclosure of an aqueous system being treated with a halogen, and no teaching that a benzotriazole or a tolyltriazole substituted with chlorine will provide corrosion protection to the cooling water system and prevent objectionable odors when halogens are present.

Werkstoffe und Korrosion, Verlag Chemie GmbM. Weinheim, DE, Vol. 30. Nr 10, Page 740. ZAK E.G. discloses the use of unsubstituted and halogen substituted benzotriazole for preventing corrosion of metals. Again however there is no disclosure of an aqueous system being treated with a halogen and no teaching that a benzotriazole or a tolyltriazole substituted with chlorine will provide corrosion protection to the cooling water system and prevent objectionable odors when halogens are present.

The nature of the reaction products when azoles are exposed to oxidizing halogens in a cooling water system is not clear. The literature teaches that a compound is formed when chlorine and tolyltriazole are combined in cooling waters, and that it responds to analytical tests for chlorine. For example, Vanderpool, et al., state that chlorine reacts reversibly with tolyltriazole to produce 1-chlorotolyltriazole. They specifically state, "presumably this compound is not itself an inhibitor." Rather, they teach that it is readily hydrolyzed to the original tolyltriazole and hypochlorous acid so that free tolyltriazole becomes available for corrosion inhibition ("Improving the Corrosion Inhibitor Efficiency of Tolyltriazole in the Presence of Chlorine and Bromine", NACE Corrosion/87, Paper No. 157 (1987)). Hollander and May were able to isolate 1-chlorotolyltriazole from stored, more highly concentrated solutions, but they also teach that "at low concentrations (less than 10 mg/L) rapid hydrolysis made it impossible to isolate the chloro adducts." "The Chemistry of Azole Copper Corrosion Inhibitors in Cooling Water", NACE-Corrosion Vol. 41, No. 1, pp. 39-45, January 1995. Thus, the art teaches that solutions of tolyltriazole with hypochlorite used to treat cooling waters are not readily distinguishable as different from a simple mixture of these components.

In contrast, the present authors have shown that a corrosion inhibitor obtainable by
i) dissolution of a benzotriazole in an aqueous acetic acid, thereafter adding a halogenating agent, recovering a resulting solid and dissolving recovered solid in an alcohol or strong alkaline solution to form ex situ said corrosion inhibitor, or by
ii) the addition of a halogenating agent to an aqueous slurry of a benzotriazole at alkaline pH to form, ex situ, said corrosion inhibitor, does not respond to analytical tests for chlorine, despite extended boiling.

In solutions of said corrosion inhibitor there is also the surprising absence of a very characteristic odor that is present whenever tolyltriazole and hypochlorite are combined in cooling waters

There are also references in the literature to 5-chlorobenzatriazole (i.e., CAS number [94-97-3]). In "The Water Drop", Volume I No. 2, 1985, Puckorius & Associates state that chlorinated tolyltriazole is effective as a corrosion inhibitor and cite R.P. Carr as a reference. However, they imply a chlorination of the benzene ring as opposed to the azole ring

Further, a literature review of published work by Carr indicates that he actually teaches that reactions between tolyltriazole and chlorine do not occur under cooling water conditions ("The Performance of Tolyltriazole in the Presence of Sodium Hypochlorite Under Simulated Field Conditions", NACE Corrosion/83 Paper No. 283, 1983). In this Corrosion/83 paper, Carr does discuss the inhibiting action of a chloroazole but it is a reference to earlier literature and specifically to the action of 5-chlorobenzotriazole and related aryl substituted chlorinated azoles in sulfuric acid solutions ("Effects of Substituted Benzotriazole on the Electrochemical Behavior of Copper in H₂SO₄", Wu et al., Corrosion, Volume 37, No. 4, 223 (1981)). Since the 1985 Puckorius reference, there has been widespread use of tolyltriazole in chlorinated cooling systems with well established performance difficulties, indicating a continuing, unsolved problem in the art.

Other problems are well-known when tolyltriazole and oxidizing halogens are combined in cooling waters. These include a loss in the extent of precipitation of transition metal ions such as copper, thus leading to improved transport and galvanic corrosion, a change in the response of the standard spectrophotometric test for tolyltriazole, leading to unintentional overfeed, and the objectionable odor mentioned above. This odor can be sensed even when the cooling water originally contained 1 ppm tolyltriazole, or less. Since cooling water often passes over cooling towers, evaporation and drift release the objectionable odor to the local environment.

The present authors believe that although the molecularity of the odorous material has not been identified, it may be the key in understanding the differences between true solutions of 1-chlorotolyltriazole in contrast to mixtures of tolyltriazole and hypochlorite: the odorous material is probably an intermediate in stepwise reactions leading to 1-chlorotolyltriazole, that it forms reversibly with tolyltriazole, and that it is absent when the reaction is driven to completion, i.e., tolyltriazole + OCI⁻ ↔ (intermediate)→1-chlorotolyltriazote. The present inventors have found no evidence of reversion of 1-chlorotolyltriazole to either the odorous intermediate or to tolyltriazole. Nor is there any evidence of reactions between hypochlorite and 1-chlorotolyltriazole in dilute aqueous solutions.

### SUMMARY OF THE INVENTION

The present inventors have discovered that corrosion inhibitors obtainable by
i) dissolution of benzotriazole, a butylbenzotriazole or a tolyltriazole in aqueous acetic acid, thereafter adding a chlorinating agent while maintaining the temperature at about 20°C, recovering a resulting solid and dissolving recovered solid in an alcohol or strong alkaline solution to form ex situ said corrosion inhibitor, or by
ii) the addition of a chlorinating agent to an aqueous slurry of benzotriazole, a butylbenzotriazole or a tolyltriazole at alkaline pH and maintaining the temperature at about 45°C, to form, ex situ, said corrosion inhibitor are more effective than tolyltriazole in inhibiting corrosion in aqueous systems being treated with a halogen. The corrosion inhibitors used in the method of the invention are substantially more effective than tolyltriazole in the presence of chlorine. Furthermore, when the corrosion inhibitors are exposed to chlorine in this method of the invention, an objectionable odor does not form and the quantity of chlorine that is required to produce a residual in the aqueous system is reduced.

According to the present invention there is provided a method of inhibiting corrosion of metal surfaces contacted by an aqueous system being treated with a halogen characterized by adding to said aqueous system a corrosion inhibitor obtainable by
i) dissolution of benzotriazole, a butylbenzotriazole or a tolyltriazole in aqueous acetic acid, thereafter adding a chlorinating agent while maintaining the temperature at about 20°C, recovering a resulting solid and dissolving recovered solid in an alcohol or strong alkaline solution to form ex situ said corrosion inhibitor, or by
ii) the addition of a chlorinating agent to an aqueous slurry of benzotriazole, a butylbenzotriazole or a tolyltriazole at alkaline pH and maintaining the temperature at about 45°C, to form, ex situ, said corrosion inhibitor.

### DESCRIPTION OF THE PREFERRED EMBODIMENTS

The present inventors have discovered that the corrosion inhibitors (prepared ex situ) are more effective than tolyltriazole in inhibiting corrosion in aqueous systems being treated with a halogen. The corrosion inhibitors are substantially more effective corrosion inhibitors than tolyltriazole in the presence of chlorine. The efficacy of the present invention is surprising given the prior knowledge that chlorination of an azole treated system leads to degradation of corrosion inhibition performance. Furthermore, the corrosion inhibitors used in the method of the present invention are not subject to the formation of objectional odors when exposed to chlorine as is tolyltrazole, the quantity of chlorine that is required to produce a residual in the aqueous system is notably reduced in comparison to systems treated with tolyltriazole, and the treatment is effective in the presence of sulfide ions.

The preferred azole for the preparation of the compound inhibitors used in the invention is tolyltriazole.

The ex situ preparation of the corrosion inhibitors may involve e.g. reactions with hypochlorite, N-chlorosuccinimide, and other chlorinating agents. One method is through the reaction of tolyltriazole with hypochlorite, in which case the final reaction mixture is an alkaline solution that can be used with or without further modification. An alternative method involves the reaction of tolyltriazole with hypochlorite in acetic acid solutions, (i.e., hypochlorous acid) and then isolated as a solid. For convenience of application, the solid can be redissolved in alcohols such as methanol or 2-propanol, aqueous solutions of alcohols or strong alkaline solutions such as sodium hydroxide or potassium hydroxide.

In treating an aqueous system in accordance with the present invention, the corrosion inhibitor is preferably fed continuously to the water. A preferred treatment concentration ranges from about 0.5 to 10 parts per million, most preferably at about 3 parts per million. Continuous feed is not, however, a requirement. The corrosion inhibitor can be fed at a concentration sufficient to form a protective film and thereafter feed can be discontinued for extended periods of time.

The treatment of the present invention can be used in combination with other corrosion and/or deposit inhibiting treatments known in the art including but not limited to phosphates, phosphonates acrylic homo- and copolymers, chelants, and oximes.

The present invention will now be further described with reference to a number of specific examples which are to be regarded solely as illustrative, and not as restricting the scope of the present invention. Examples 1 to 3 and 7 illustrate the preparation of compounds used in the method of the invention.

### Examples

### Example 1

The preparation of the solid samples was as follows:

Tolyltriazole (hereinafter TTA) (30g, 0.225 mol) was dissolved in aqueous acetic acid (60 mL. 1:1 ratio) by heating to 32°C. Sodium hypochlorite (366 g, 5.25% sodium hypochlorite as a bleach solution) was added while maintaining the reaction temperature at - 20°C. Following the addition, the reaction mixture was stirred at room temperature for 24 hours. A sticky precipitate formed during this time. The solid was filtered and taken into methylene chloride. The solid that did not dissolve was filtered and the methylene chloride was removed to obtain a yellow solid which was the corrosion inhibitor with minor amounts of di-CI-TTA. Unless noted, this yellow solid was used in the following Examples.

### Example 2

A slurry of TTA (50 g, 0.376 mol) in 25 g of water was warmed to 35°C. Sodium hypochlorite (27.9 g, 0.376 mol, added as 226.8 g of a 12.3% sodium hypochlorite solution) was added over a period of 2 hours. After the addition, the reaction was kept at 45°C for one hour. During the addition the pH of the reaction mixture increased to 12 and the solids dissolve. The final product was analyzed by ¹H and ¹³C NMR and LC-UV and found to be composed of 81.9% CI-TTA: 8.8% residual TTA, and 9.3% di-CI-TTA based on the relative areas in the UV spectra.

On dilution to 1 to 100 ppm azole, with or without pH adjustment to about 7.2, there was no odor from the solution of the treatment material of the present invention.

### Example 3

In the schemes below, TTA was present at 100 ppm, in contrast to Example 2 where the initial slurry contained about 200,000 ppm. "x" derotes a stoichiometric ratio.

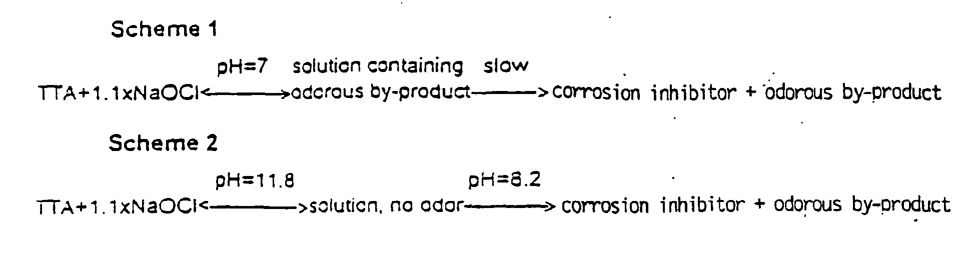

Corrosion tests were carried out using a simulated cooling water solution which contained 500 ppm Ca, 250 ppm Mg, 25 ppm M-alkalinity, 15 ppm o-PO₄, 3 ppm tetrapotassium pyrophosphate, 10 ppm of a 3:1, low molecular weight, acrylic acid/allyl 2-hydroxypropyl sulfonate ether copolymer, 2.4 ppm HEDP. The test material was the corrosion inhibitor prepared in accordance with Example 1 and was compared with TTA. The solution was maintained at 49°C by an admiralty brass heater tube and of a pH of 7.2 with a blended mixture of air and carbon dioxide for 20 hours, and the pH maintained at 7.2 with a blended mixture of air and carbon dioxide at 49°C for 20 hours.

A solution of sodium hypochlorite was added after 20 hours and continued for an additional 72 hours. The feed rate of the sodium hypochlorite was controlled to produce a "chlorine residual" of about 0.1 to 0.3 ppm as Cl₂ using a standard DPD spectrophotometric test on the recirculating water. The corrosion inhibitor prepared in accordance with Example 1 was fed continuously at 3 ppm during the experiments and was supplied by dissolving the solid in potassium hydroxide solution and then diluting it into the feedwater for the system. For the experiment with the corrosion inhibitor prepared in accordance with Example 1 the feed rate of the sodium hypochlorite was about 30% of that required for TTA. For TTA, the characteristic odor was detected immediately after the first hypochlorite was added. With the corrosion inhibitor prepared in accordance with Example 1; there was no odor upon initiating hypochlorite addition, and only a trace was sensed just prior to concluding the four day run. Each experiment was duplicated: once with an admiralty brass heated tube, and once with a low carbon steel heated tube. Corrosion rates were measured from admiralty and LCS working electrodes, and by weight changes of admiralty and LCS coupons. Rates for the coupons were measured for the initial day of each run, and a "differential" rate was calculated for the remaining days of the run by offsetting the initial rate from the overall rate.

Electrochemical corrosion rates were measured using admiralty brass (ADM) and low carbon steel (LCS) working electrodes. All tests also had both admiralty and LCS coupons in contact with the solution.

**TABLE I**

| CRM Corrosion Rates: Values at 90 hour mark in mpy (figures in brackets are in mm/year) | | |
|---|---|---|
| LCS Heated Surface | | |
| | A | B |
| LCS | 0.5 (0.0127) | 2.3 (0.0584) |
| ADM | 0.06 (0.0152) | 0.02 (0.00051) |

**TABLE II**

| Gravimetric Corrosion Rates (mpy) | | |
|---|---|---|
| LCS Heated Surface | | |
| | A | B |
| Day 2 to 4 LCS | 1.1 (0.0279) | 2.6 (0.0660) |
| Day 4 LCS (diff) | 0.4 (0.0102) | 1.4 (0.0355) |
| Day 2 to 4 ADM | 1.1 (0.0279) | 1.2 (0.0305) |
| Day 4 ADM (diff) | 0.15 (0.0038) | 0.85 (0.0216) |

| | | |
|---|---|---|
| A = corrosion inhibitor prepared in accordance with Example 1 | | |
| B = TTA | | |

### Example 5

Admiralty brass corrosion coupons and working electrodes were coated with a sulfide layer by exposing the metal to a sodium sulfide solution for 18 hours. These samples were rinsed and dried. Corrosion tests were carried out in aqueous solutions in stirred beakers containing 500 ppm Ca, 250 ppm Mg, 25 ppm M-alkalinity, 15 ppm o-PO₄, 3 ppm tetrapotassium pyrophosphate, 10 ppm of a 3:1, low molecular weight, acrylic acid/allyl 2-hydroxypropyl sulfonate ether copolymer, 2.4 ppm HEDP, and the pH was maintained at 7.2 with a blended mixture of air and carbon dioxide at 49°C for 18 hours. Electrochemical corrosion rates were measured using admiralty brass or low carbon steel working electrodes. All tests also had both admiralty and LCS coupons in contact with the solution.

Each solution was tested with and without addition cf sodium hypochlorite (added after 1 hour exposure). In a separate, but otherwise identical experiment, clean low carbon steel working electrodes were used in place of the sulfide-exposed admiralty brass, but the sulfide-exposed brass coupons were present as a source of copper. At the conclusion of the experiment, a sample of the supematant solution was taken and analyzed for copper. Analyses were taken with and without filtration through a 0.2 micron membrane filter.

**TABLE III**

| Azole | NaOCI (ppm) | | Admiralty Brass Corrosion Rate | Low Carbon Steel Corrosion Rate | Copper Unfiltered | (ppm). Filtered |
|---|---|---|---|---|---|---|
| none | | 0 | 1.01 (0.0256) | 5.4 (0.1372) | 0.354 | 0.103 |
| 3 ppm TTA | | 0 | 0.07 (0.0018) | 1.2 (0.0305) | 0.014 | 0.014 |
| 3 ppm corrosion inhibitor prepared corrosion inhibitor in accordance with Example 1 | | 0 | 0.06 (0.00152) | 1.0 (0.0254) | 0.005 | 0.004 |
| | | | 0.05 (0.00127 | | | |
| none | | 2.0 | 2.09 (0.0531) | 5.2 (0.1321) | 0.417 | 0.059 |
| 3 ppm | | 2.0 | 0.45 (0.0114) | 2.6 (0.0660) | 0.133 | 0.066 |
| 3 ppm corrosion inhibitor prepared in accordance with Example 1 | | 2.0 | 0.13 (0.0033) | 1.7 (0.0432) | 0.086 | 0.039 |

All corrosion rates are in mpy with mm/year in brackets.

### Example 6

A synthetic sea water was formulated from deionized water plus 1010 ppm Ca as CaCO₃, 5226 ppm Mg (as CaCO₃), 18971 ppm Cl, 2660 ppm SO₄, 117 ppm M-alkalinity (as CaCO₃), 5 ppm azole (see below), and the pH was maintained at 7.8 with a blended mixture of air and carbon dioxide at 38°C.

Admiralty brass electrodes were exposed to this medium for 1 hour and then they were transferred to identical water with no azole present. Electrochemical corrosion rates were measured for 18 hours.

**Table IV**

| Azole | Mean Electrochemical Corrosion Rate (in mpy with mm/year in brackets) |
|---|---|
| Benzotriazole | 40 (1.016) |
| 5-Butylbenzotriazole | 15 (0.381) |
| Tolyltriazole | 6 (0.1524) |
| corrosion inhibitor prepared in accordance with Example 1 | 3.2 (0.0813) |

### Example 7

Sodium hypochlorite (12, 2%, 204.9g, 0.336 mol) was added over 90 minutes to a stirring slurry of benzotriazole (40g, 0.336 mol) in 30g of water at room temperature. Following the addition, the reaction mixture was held at 45-50°c for one hour. Upon cooling, a precipitate formed. A clear yellow solution of a corrosion inhibitor was obtained after adjusting the pH to 11.

### Example 8

The method of Example 5 was followed, using samples from Examples 2 and 7 at 1 to 4 ppm total actives. The following were the 18 hour averaged electrochemical corrosion rates.

**TABLE V**

| Azole | Conc. (ppm) | NaOCl | Average Corrosion Rate (in mpy with mm/year in brackets) |
|---|---|---|---|
| corrosion inhibitor | 1 | none | 0.21 (0.00533) |
| prepared in accordance with | 2 | | 0.09 (0.00229). |
| Example 7 | 4 | | 0.03 (0.00076) |
| corrosion inhibitor | 1 | none | 0.14 (0.00355) |
| prepared in accordance with | 2 | | 0.09 (0.00229) |
| Example 2 | 4 | | 0.08 (0.00203) |
| TTA | 1 | none | 0.13 (0.0033) |
| | 2 | | 0.14 (0.00355) |
| | 4 | | (n/a) |
| corrosion inhibitor | 1 | 2 ppm | 0.55 (0.01397) |
| prepared in | 2 | | 0.25 (0.00635) |
| accordance with | 4 | | 0.09 (0.00229) |
| Example 7 | | | |
| corrosion inhibitor | 1 | 2 ppm | 0.58 (0.1473) |
| prepared in | 2 | | 0.24 (0.0061) |
| accordance with | 4 | | 0.09 (0.00229) |
| Example 2 | | | |
| TTA | 1 | 2 ppm | (n/a) |
| | 2 | | 0.45 (0.01143) |
| | 4 | | 0.27 (0.00686) |

The above examples show that the ex situ prepared materials of the present invention are effective corrosion inhibitors even in the presence of chlorine.

## Claims

1. A method of inhibiting corrosion of metal surfaces contacted by an aqueous system being treated with a halogen **characterized by** adding to said aqueous system a corrosion inhibitor obtainable by
i) dissolution of benzotriazole, a butylbenzotriazole or a tolyltriazole in aqueous acetic acid, thereafter adding a chlorinating agent while maintaining the temperature at about 20°C, recovering a resulting solid and dissolving recovered solid in an alcohol or strong alkaline solution to form ex situ said corrosion inhibitor, or by
ii) the addition of a chlorinating agent to an aqueous slurry of benzotriazole, a butylbenzotriazole or a tolyltriazole at alkaline pH and maintaining the temperature at about 45°C, to form, ex situ, said corrosion inhibitor.

2. A method as claimed in claim 1, wherein sulfide ions are present in said aqueous system.

3. A method as claimed in claim 1 or 2, wherein said corrosion inhibitor is obtainable by dissolution of a tolyltriazole in aqueous acetic acid, thereafter adding a chlorinating agent while maintaining the temperature at about 20°C, recovering a resulting solid and dissolving the recovered solid in an alcohol or strong alkaline solution to form ex situ said corrosion inhibitor.

4. A method as claimed in any one of the preceding claims, wherein said corrosion inhibitor is added to said aqueous system at a concentration of greater than 0.5 parts per million.

5. A method as claimed in any one of the preceding claims, wherein said corrosion inhibitor is added to said aqueous system at a concentration of from 0.5 parts per million to 10 parts per million.

6. A method as claimed in any one of the preceding claims, wherein a corrosion inhibiting layer is formed on said metal surfaces by said corrosion inhibitor.

7. A method as claimed in any one of the preceding claims, wherein chlorine demand, in said aqueous system, to inhibit microbiological growth is reduced by said corrosion inhibitor.

8. A method as claimed in any one of the preceding claims, wherein copper ion transport in said aqueous system is inhibited by said corrosion inhibitor.

9. A method as claimed in any one of the preceding claims, wherein said corrosion inhibitor is used in combination with an additional corrosion and/or deposit inhibiting treatment.

10. A method as claimed in claim 9, wherein said additional corrosion and/or deposit inhibiting treatment is selected from phosphates, phosphonates, acrylic homo- and copolymer, chelants and oximes.

11. A method as claimed in claims 1 or 2, wherein said corrosion inhibitor is obtainable by the addition of a chlorinating agent to an aqueous slurry of tolyltriazole at alkaline pH and maintaining the temperature at about 45°C, to form ex situ, said corrosion inhibitor.

## Patentansprüche

1. Verfahren zur Hemmung der Korrosion von Metalloberflächen, die mit einem wässrigen System, das mit einem Halogen behandelt ist, in Kontakt sind, das durch die Zugabe eines Korrosionshemmers zu genanntem wässrigen System **gekennzeichnet** ist, der durch
i) Auflösung von Benzotriazol, einem Butylbenzotriazol oder einem Tolyltriazol in wässriger Essigsäure, dann durch Zugabe eines chlorierenden Agens unter Aufrechterhaltung der Tempereatur bei ca. 20°C, Gewinnung eines entstehenden Feststoffes und Auflösung des gewonnenen Feststoffes in einem Alkohol oder einer stark alkalischen Lösung zur ex-situ-Bildung von genanntem Korrosionshemmer, oder durch
ii) die Zugabe eines chlorierenden Agens zu einer wässrigen Aufschlämmung von Benzotriazol, einem Butylbenzotriazol oder einem Tolyltriazol bei alkalischem pH und die Aufrechterhaltung der Temperatur bei ca. 45°C zur ex-situ-Bildung von genanntem Korrosionshemmer erhalten werden kann.

2. Verfahren nach Anspruch 1, worin in genanntem wässrigen System Sulfidionen gegenwärtig sind.

3. Verfahren nach Anspruch 1 oder 2, worin genannter Korrosionhemmer durch Auflösung eines Tolyltriazols in wässriger Essigsäure, dann durch Zugabe eines chlorierenden Agens unter Aufrechterhaltung der Tempereatur bei ca. 20°C, Gewinnung eines entstehenden Feststoffes und Auflösung des gewonnenen Feststoffes in einem Alkohol oder einer stark alkalischen Lösung zur ex-situ-Bildung von genanntem Korrosionshemmer erhalten werden kann.

4. Verfahren nach einem der vorstehenden Ansprüche, worin genannter Korrosionshemmer zu genanntem wässrigen System bei einer Konzentration von größer als 0,5 parts per million zugegeben wird.

5. Verfahren nach einem der vorstehenden Ansprüche, worin genannter Korrosionshemmer zu genanntem wässrigen System bei einer Konzentration von 0,5 parts per million bis 10 parts per million zugegeben wird.

6. Verfahren nach einem der vorstehenden Ansprüche, worin eine korrosionshemmende Schicht auf genannten Metalloberflächen durch genannten Korrosionshemmer gebildet wird.

7. Verfahren nach einem der vorstehenden Ansprüche, worin der Bedarf an Chlor in genanntem wässrigen System zur Hemmung von mikrobiologischem Wachstum durch genannten Korrosionshemmer reduziert ist.

8. Verfahren nach einem der vorstehenden Ansprüche, worin der Transport von Kupferionen in genanntem wässrigen System durch genannten Korrosionshemmer gehemmt ist.

9. Verfahren nach einem der vorstehenden Ansprüche, worin genannter Korrosionshemmer zusammen mit einer zusätzlichen, die Korrosion und/oder Ablagerung hemmenden Behandlung verwendet wird.

10. Verfahren nach Anspruch 9, worin genannte zusätzliche, die Korrosion und/oder Ablagerung hemmende Behandlung aus Phosphaten, Phosphonaten, acrylischem Homo- und Copolymer, Chelatbildnern und Oximen ausgewählt wird.

11. Verfahren nach Anspruch 1 oder 2, worin genannter Korrosionshemmer durch die Zugabe eines chlorierenden Agens zu einer wässrigen Aufschlämmung von Tolyltriazol bei alkalischem pH und die Aufrechterhaltung der Temperatur bei ca. 45°C zur ex-situ-Bildung von genanntem Korrosionshemmer erhalten werden kann.

## Revendications

1. Procédé d'inhibition de la corrosion de surfaces métalliques mises au contact d'un système aqueux traité avec un halogène, **caractérisé par** l'addition audit système aqueux d'un inhibiteur de la corrosion pouvant être obtenu par
i) la dissolution de benzotriazole, d'un butylbenzotriazole ou d'un tolyltriazole dans de l'acide acétique aqueux, après quoi on ajoute un agent de chloration tout en maintenant la température à environ 20°C, on récupère un solide résultant et on dissout le solide récupéré dans un alcool ou une solution alcaline forte en vue de former ex situ ledit inhibiteur de corrosion, ou par
ii) l'addition d'un agent de chloration à une bouillie aqueuse de benzotriazole, d'un butylbenzotriazole ou d'un tolyltriazole à un pH alcalin et on maintient la température à environ 45°C en vue de former ex situ ledit inhibiteur de corrosion.

2. Procédé selon la revendication 1, dans lequel des ions sulfure sont présents dans ledit système aqueux.

3. Procédé selon la revendication 1 ou 2, dans lequel ledit inhibiteur de corrosion peut être obtenu par dissolution d'un tolyltriazole dans de l'acide acétique aqueux, après quoi on ajoute un agent de chloration tout en maintenant la température à environ 20°C, on récupère un solide résultant et on dissout le solide récupéré dans un alcool ou une solution alcaline forte en vue de former ex situ ledit inhibiteur de corrosion.

4. Procédé selon l'une quelconque des revendications précédentes, dans lequel ledit inhibiteur de corrosion est ajouté audit système aqueux à une concentration supérieure à 0,5 partie par million.

5. Procédé selon l'une quelconque des revendications précédentes, dans lequel ledit inhibiteur de corrosion est ajouté audit système aqueux à une concentration allant de 0,5 partie par million à 10 parties par million.

6. Procédé selon l'une quelconque des revendications précédentes, dans lequel une couche inhibitrice de corrosion est formée sur lesdites surfaces métalliques par ledit inhibiteur de corrosion.

7. Procédé selon l'une quelconque des revendications précédentes, dans lequel la demande en chlore pour inhiber la croissance microbiologique dans ledit système aqueux est réduite par ledit inhibiteur de corrosion.

8. Procédé selon l'une quelconque des revendications précédentes, dans lequel le transport d'ions cuivre dans ledit système aqueux est inhibé par ledit inhibiteur de corrosion.

9. Procédé selon l'une quelconque des revendications précédentes, dans lequel ledit inhibiteur de corrosion est utilisé en association avec un traitement d'inhibition de corrosion et/ou dépôt supplémentaire.

10. Procédé selon la revendication 9, dans lequel ledit traitement d'inhibition de corrosion et/ou dépôt supplémentaire est choisi parmi les phosphates, les phosphonates, les homo- et copolymères acryliques, les chélateurs et les oximes.

11. Procédé selon les revendications 1 ou 2, dans lequel ledit inhibiteur de corrosion peut être obtenu par l'addition d'un agent de chloration à une bouillie aqueuse de tolyltriazole à un pH alcalin et on maintient la température à environ 45°C en vue de former ex situ ledit inhibiteur de corrosion.
